# EUROPÄISCHE PATENTSCHRIFT

(11) **EP 0 453 593 B1**
(45) Veröffentlichungstag und Bekanntmachung des Hinweises auf die Patenterteilung: **05.10.1994**
(21) Anmeldenummer: 90107746.1
(22) Anmeldetag: 24.04.1990
(51) Int. Cl.: A01M 17/00, A23B 9/10, A23B 7/055

(54) **Entwesung**
Eradicating pests
Extermination des vermines

(43) Veröffentlichungstag der Anmeldung: 30.10.1991
(73) Patentinhaber: SAUERSTOFFWERK FRIEDRICH GUTTROFF GMBH, D-97877 Wertheim (DE)
(72) Erfinder: Tallafus, Ottmar, D-6967 Buchen 1 (DE)
(74) Vertreter: Trappenberg, Hans

(56) Entgegenhaltungen:
- EP-A- 0 052 574
- EP-A- 0 320 012
- CH-A- 365 274
- DE-A- 2 602 034
- DE-A- 3 445 990
- DE-A- 3 822 544
- FR-A- 2 567 722
- GB-A- 518 118
- JP-A-50 058 210
- US-A- 4 322 910

## Beschreibung

Die Erfindung betrifft ein Verfahren zum Entwesen von trockenen Drogen (trockene Gewürze, Tees, Heildrogen etc.) durch Einleiten von Gas in einen die Drogen aufnehmenden geschlossenen Behälter zum Abtöten sowohl der adulten Tiere, wie auch deren Larven und Eier.

Einem Aufsatz der Autoren Egon Stahl, G. Rau und H. Adolphi in der Zeitschrift "Die Pharmazeutische Industrie" (Pharm. Ind. 47, 5, 528 bis 530 (1985)) ist folgendes zu entnehmen:
"Drogen sind immer mehr oder weniger stark von Schadinsekten, Larven oder Eiern befallen. Dies führt insbesondere bei einer längeren Lagerung zu erheblichen Verlusten.

Als Entwesungsmittel zum Abtöten von Insekten, Larven und Eiern werden Phosphin, Blausäure und Methylbromid verwendet. Diese Gase sind sowohl wegen ihrer Giftigkeit, als auch wegen den Restmengen, die in den Drogen verbleiben und den entstehenden Metaboliten in der Verwendung stark eingeschränkt. Das bis vor kurzem sehr häufig verwendete und inzwischen als kanzerogen eingestufte Ethylenoxid ist sowohl als Schädlingsbekämpfungsmittel als auch zur Entwesung verboten.

Andere Verfahren, wie etwa die Anwendung von gamma-Strahlen sowie eine Dampfsterilisation oder die Behandlung mit Alkoholdämpfen führen zu nachteiligen Veränderungen an der behandelten Droge."

Da aber, so führen die Autoren weiter aus, eine Entwesung von Drogen aus hygienischen und ökonomischen Gründen dringend erforderlich ist, wurde von ihnen nach neuen Verfahren gesucht. Als seinerzeit optimal hat sich hierbei ein Pressure/Expansion-Verfahren zur Druckentwesung von Lebensmitteln und Arzneipflanzen mit Kohlendioxid herausgestellt. Die Anwendung dieses Verfahrens geschieht, wie in einem weiteren Aufsatz der Autoren W. Pohlen, G. Rau und E. Finkenzeller in der Zeitschrift "Die Pharmazeutische Industrie" (Pharm. Ind. 51, 8, 917 - 918 (1989)) angegeben ist, folgendermaßen:
" Das zu behandelnde Material, das in seiner ursprünglichen Verpackung belassen werden kann, wird auf speziell konstruierten Transportwagen in einen Druckautoklaven eingefahren. Nach dem vollständigen Befüllen wird der Druckautoklav verschlossen. Um die Droge nicht mit Sauerstoff zu beaufschlagen, kann mittels einer Vakuumpumpe der Druckautoklav bis auf einen Restdruck von ca. 200 mbar evakuiert werden. Aus einem Vorratsbehälter strömt über eine Steuer- und Regeleinrichtung gasförmiges Kohlendioxid in die Druckkammer, bis der gewünschte Enddruck - wie dem Aufsatz weiter zu entnehmen 18 bar bis 25 bar - erreicht ist. Nach Ablauf der erforderlichen Einwirkungszeit wird das Kohlendioxid abgelassen, nach vollständigem Druckausgleich der Autoklav geöffnet und das behandelte Gut entnommen."

Die Ergebnisse dieses Pressure/Expansion-Verfahrens lassen tatsächlich eine nahezu 100%ige bis 100%ige Mortalitätsrate der Schadinsekten erkennen. Der Nachteil dieses Verfahrens ist darin zu erblicken, daß es nicht in allen Fällen die Schadinsekten, insbesondere deren Larven und Eier, vollkommen vernichtet, und daß es zu seiner Anwendung eines großen apparativen Aufwandes bedarf.

Aufgabe der Erfindung ist es damit, eine Entwesung beziehungsweise ein Verfahren zum Entwesen derartiger Drogen anzugeben, das mit Sicherheit alle Schadinsekten, wie auch deren Larven und Eier vernichtet und das auch auf wirtschaftliche Art und Weise und ohne hohen Investitionsaufwand erfordernden apparativen Aufwand auskommt. Erreicht wird dies in erfindungsgemäßer Weise dadurch, daß das Gas ein Kaltgas mit einer deutlich unter minus 20°C liegenden Temperatur, vorzugsweise Luft, Kohlendioxid oder Stickstoff in flüssigem oder gasförmigem Zustand ist.

Nicht mehr also wie bisher werden die Schadinsekten, deren Larven und Eier durch ein Druck-/Entspannungs-Verfahren vernichtet, sondern durch Kälteeinwirkung. Wie sich gezeigt hat, ist durch diese Kälteeinwirkung, wobei eine Temperatur einzuhalten ist, die unter minus 20°C liegen sollte, eine vollkommene Mortalitätsrate der Schadinsekten wie auch deren Larven und Eier zu erzielen. Dies liegt primär daran, daS durch die Kälteeinwirkung die Zellen sowohl der adulten Tiere, wie auch deren Larven und Eier zerstört werden, wie aber sicherlich auch durch die Wirkung des jeweils eingesetzten Kaltgases auf das Atemzentrum dieser Insekten. Primär dürfte jedoch, wie ausgeführt, die Kälteeinwirkung sein, was sich auch in einer Mortalitätskurve - Temperatur/Zeit - bemerkbar macht. Wie Versuche gezeigt haben, sind nach einer sehr kurzen Zeitspanne von Sekunden bis Minuten sämtliche Schadinsekten, einschließlich ihrer Larven und Eier, bei Einwirkung extrem niederer Temperaturen, beispielsweise beim Eintauchen der Drogen direkt in flüssiges Kaltgas, abgetötet. Diese vollkommene Mortalität erfordert mit steigender Temperatur eine Verlängerung der Einwirkungszeit, wobei allerdings die Mortalitätskurve asymptotisch etwa in eine minus 20°C-Linie ein- mündet, die Temperatur also nicht höher als minus 20°C steigen sollte. Ein weiteres Limit ist durch die Anpassungsfähigkeit insbesondere der Larven und Eier gegeben, die etwa bei 24 Stunden liegt. Nach dieser Zeit ist ein "Gewöhnungs"-Prozeß festzustellen, wonach durch biochemische Umstellung der Organismen eine geringere Kälteempfindlichkeit festzustellen ist.

Obwohl also der Mechanismus der Abtötung noch nicht vollkommen geklärt ist, läßt die Anwendung der erfindungsgemäßen Entwesung erkennen, daß die oben gestellten Forderungen erfüllt werden, insbesondere also eine vollkommene Mortalität erreicht wird.

Hinsichtlich der Kälteeinwirkung zum Abtöten von Schadinsekten ist auf die EP-A-052 574 zu verweisen. Das dort vorgestellte Verfahren betrifft die Behandlung frischer Kräuter und Kräutermischungen durch Zerkleinern der Kräuter und deren Kühlen. Eine Entwesung ist mit diesem Verfahren nicht beabsichtigt, obwohl sich bei dieser dort beschriebenen Behandlung wohl auch ein Entwesungseffekt einstellen wird. Das Verfahren dient dazu, die Kräuter so zu verspröden, daß sie zu einem gewünschten Granulat zerkleinert werden können. Die Aufbewahrung der Kräuter soll sodann in einer Temperatur zwischen minus 20 °C und minus 40 °C erfolgen.

Wie angeführt, hat das Verfahren nicht den Zweck die Kräuter zu entwesen, sondern sie so aufzubereiten, daß sie in granulierter Form bei Minustemperaturen gelagert werden können. Diese Aufbereitung ist bei der Entwesung trockener Gewürze, Tees, Heildrogen etc. nicht gewünscht. Vielmehr sollen diese trockenen Materialien nach Möglichkeit in der Anlieferungsverpackung belassen und in dieser Form entwest werden. Dies ist mit dem vorbekannten Verfahren nicht möglich.

Die weiter bekannte britische Patentschrift 518,118 beschäftigt sich mit der Lagerhaltung von in einem Silo untergebrachten Getreide, das mittels Kohlendioxid, gegebenenfalls zusammen mit Ethylenoxid, begast wird. Zwar wird hiermit auch die Zerstörung von Schadinsekten bezweckt, jedoch in altbekannter Weise durch Gaseinwirkung und nicht durch die Einwirkung von Kälte. Diese minimale Gaseinwirkung genügt jedoch nicht, um Insekten, Larven und Eier in den trockenen Gewürzen abzutöten. Hierfür sind deutlich giftigere Gase, wie Phosphin, Blausäure oder Methylbromid notwendig. Außerdem ist das dort beschriebene Ethylenoxid zwischenzeitlich zur Anwendung bei der Entwesung untersagt.

Auch eine französische Patentschrift 25 67722 beschreibt ein Verfahren zur Kältebehandlung von trockenem Gut, in diesem Falle von Reis, bei dem in einen Schneckenförderer flüssiges Kaltgas eingesprüht wird. Auch hier ist wiederum nicht die Abtötung von Insekten etc. im Vordergrund, sondern die Verfestigung der Reiskörner zur späteren Behandlung, so daß möglichst wenig Ausschuß beziehungsweise Bruch entsteht.

Hinsichtlich der wirtschaftlichen Seite ist zu bemerken, daß bereits durch die Anwendung der drucklosen Behälter eine starke Verbilligung der zu verwendenden Apparatur stattfindet. Außerdem könnte nach der Erfindung durchaus kontinuierlich entwest werden, dann, wenn statt des Behälters ein Kühltunnel oder auch eine, zweckmäßigerweise mit flüssigem Kaltgas zu beschickende Kühlförderschnecke eingesetzt wird. Hinsichtlich der Wirtschaftlichkeit ist auch zu beachten, daß der Gasart nur eine sekundäre Bedeutung zukommt, also das jeweils am wirtschaftlichsten zu erhaltende beziehungsweise einsetzbare Kaltgas verwendet werden kann. Zu beachten ist schließlich auch die mögliche Verkürzung der Entwesungszeit.

Ein Verfahren zum Durchführen des Entwesens wird nach der Erfindung wie folgt vorgeschlagen:
a) Einbringen der in Anlieferungsverpackung belassenen Drogen in einen wärmeisolierten Behälter,
b) Schließen des Behälters und Einblasen/Einlassen des Kaltgases,
c) Belassen der Drogen im Behälter, bis sich auch im Kern der Drogenpackungen die gewünschte Minustemperatur eingestellt hat,
d) Austragen der entwesten Drogen.

Wie oben schon angeführt, kann statt des Behälters auch eine Schnellfrostzelle oder ein Kühltunnel eingesetzt werden oder ein mit flüssigem Kaltgas gefülltes Tauchbad beziehungsweise eine entsprechende Kühlförderschnecke. Wird ein Behälter eingesetzt, so sollte zweckmäßigerweise eine am Behälter angebrachte Ausblasklappe (Auslaßventil) nach dem durch das Einblasen/Einlassen des Kaltgases bewirkten Ausblasen der Luft so weit geschlossen werden, daß sich im Behälter ein geringer Überdruck, der das Einströmen von Luft verhindert, aufbaut.

Aus der beigefügten Kurve ist als Anhalt die Einwirkungszeit des Kaltgases in Abhängigkeit von der Temperatur im Kern der Drogenpackung dargestellt. Hieraus ist beispielsweise zu erkennen, daß eine Entwesung schon nach im Sekundenbereich liegenden Zeiten bei direkter Einwirkung von flüssigem Kaltgas stattgefunden hat, daß jedoch bei einer Kerntemperatur der Drogenpackung von minus 25°C eine Einwirkungszeit von etwa vier Stunden notwendig ist.

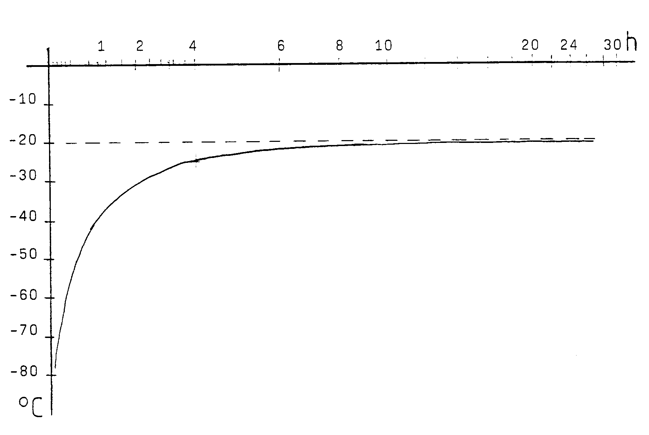

## Patentansprüche

1. Verfahren zum Entwesen von trockenen Drogen (trockene Gewürze, Tees, Heildrogen etc.) durch zeitweises Durchfrieren mittels kalter Gase in einem geschlossenen Behälter, zum Abtöten sowohl der adulten Tiere, wie auch deren Larven und Eier,
dadurch gekennzeichnet,
daß das kalte Gas Kaltgas ist, das nach Einleiten in den geschlossenen Behälter zu einer unter minus 25°C liegenden Gastemperatur führt und daß die Drogen solange, jedoch maximal 24 Stunden, in dieser kalten Gasatmosphäre verbleiben, bis sich auch im Kern der Drogen zumindest diese Temperatur eingestellt hat, wobei das Zeit/Temperaturverhältnis gemäß einer Kurve verläuft, die von einem Punkt sehr niedriger Temperaturen wie zum Beispiel minus 80°C bei Einwirkungszeiten von wenigen Sekunden bis Minuten asymptotisch für längere Einwirkungszeiten in eine etwa bei minus 20°C liegende Temperaturlinie einmündet.

2. Verfahren zum Entwesen nach Anspruch 1,
dadurch gekennzeichnet,
daß das Kaltgas flüssiges und/oder gasförmiges Kohlendioxid (LCO2/CO2) ist.

3. Verfahren zum Entwesen nach Anspruch 1,
dadurch gekennzeichnet,
daß das Kaltgas flüssiger und/oder gasförmiger Stickstoff (LN2/N2) ist.

4. Verfahren zum Entwesen nach Anspruch 1,
dadurch gekennzeichnet,
daß das Kaltgas flüssige und/oder gasförmige Luft ist.

5. Verfahren zum Entwesen nach einem der Ansprüche 1 bis 4,
gekennzeichnet
durch folgende Verfahrensschritte:
a) Einbringen der in Anlieferungsverpackung belassenen Drogen in einen wärmeisolierten Behälter,
b) Schließen des Behälters und Einblasen/Einlassen des Kaltgases,
c) Belassen der Drogen im Behälter, bis sich auch im Kern der Drogenpackungen die gewünschte Minustemperatur eingestellt hat,
d) Austragen der entwesten Drogen.

6. Verfahren zum Entwesen nach Anspruch 5,
dadurch gekennzeichnet,
daß der Behälte eine Schnellfrostzelle ist.

7. Verfahren nach Anspruch 5 ,
dadurch gekennzeichnet,
daß der Behälter ein Kühltunnel ist.

8. Verfahren nach Anspruch 5,
dadurch gekennzeichnet,
daß der Behälter ein mit Flüssig-Kaltgas gefülltes Tauchbad ist.

9. Verfahren nach Anspruch 5,
dadurch gekennzeichnet,
daß der Behälter eine in ihrem Einfüllbereich mit Flüssig-Kaltgas gefüllte Kühlförderschnecke ist.

10. Verfahren nach Anspruch 5,
dadurch gekennzeichnet,
daß eine am Behälter angebrachte Ausblasklappe (Auslaßventil) nach dem durch das Einblasen/Einlassen des Kaltgases bewirkten Ausblasen der Luft so weit geschlossen wird, daß sich im Behälter ein geringer Überdruck aufbaut.

## Claims

1. A method of sterilizing dry drugs (dry herbs, teas, medical drugs etc) by temporary freezing by means of cold gases in a closed container, for killing off both the adult animals and also their larvae and eggs, characterised in that the cold gas is cold gas which after introduction into the closed container results in a gas temperature which is below minus 25°C and that the drugs remain in that cold gas atmosphere until at least that temperature also obtains in the core of the drugs, but for a maximum of 24 hours, the time/temperature ratio being in accordance with a curve which from a point of very low temperatures such as for example minus 80°C with periods of action of a few seconds to minutes goes asymptotically for longer periods of action into a temperature line which is approximately at minus 20°C.

2. A sterilizing method according to claim 1 characterised in that the cold gas is liquid and/or gaseous carbon dioxide (LCO2/CO2).

3. A sterilizing method according to claim 1 characterised in that the cold gas is liquid and/or gaseous nitrogen (LN2/N2).

4. A sterilizing method according to claim 1 characterised in that the cold gas is liquid and/or gaseous air.

5. A sterilizing method according to one of claims 1 to 4 characterised by the following method steps:
a) introducing the drugs which are left in their delivery packaging into a heat-insulated container,
b) closing the container and blowing in/letting in the cold gas,
c) leaving the drugs in the container until the desired minus temperature also obtains in the core of the drug packs, and
d) removing the sterilized drugs.

6. A sterilizing method according to claim 5 characterised in that the container is a quick-freeze cell.

7. A method according to claim 5 characterised in that the container is a cooling tunnel.

8. A method according to claim 5 characterised in that the container is an immersion bath filled with liquid cold gas.

9. A method according to claim 5 characterised in that the container is a cooling conveyor screw filled in its charging region with liquid cold gas.

10. A method according to claim 5 characterised in that a blow-out flap (outlet valve) disposed on the container, after the air is blown out by the cold gas being blown in/let in, is closed to such an extent that a slightly increased pressure is built up in the container.

## Revendications

1. Procédé d'extermination des vermines dans des produits médicinaux séchés (épices séchées, tisanes, drogues médicinales, etc. ), par congélation temporaire dans un récipient clos, au moyen de gaz froids, en vue de tuer tant les individus adultes que leurs larves et oeufs,
caractérisé par le fait
que le gaz froid est un gaz refroidi impliquant, après introduction dans le récipient clos, une température gazeuse inférieure à moins 25°C ; et par le fait que les produits médicinaux demeurent dans cette atmosphère gazeuse froide, au maximum 24 heures cependant, jusqu'à ce qu'au moins cette température se soit établie également au coeur des produits médicinaux, le rapport temps/température étant conforme à une courbe qui, à partir d'un point de températures très basses, comme par exemple moins 80°C pour des temps d'action de quelques secondes à quelques minutes seulement, débouche asymptotiquement, pour des temps d'action plus longs, sur une ligne de température d'environ moins 20°C.

2. Procédé d extermination des vermines, selon la revendication 1,
caractérisé par le fait
que le gaz refroidi est du dioxyde de carbone liquide et/ou gazeux (LCO2/CO2).

3. Procédé d extermination des vermines selon la revendication 1,
caractérisé par le fait
que le gaz refroidi est de l'azote sous forme liquide et/ou gazeuse (LN2/N2).

4. Procédé d'extermination des vermines, selon la revendication 1,
caractérisé par le fait
que le gaz refroidi est de l'air sous forme liquide et/ou gazeuse.

5. Procédé d extermination des vermines, selon l'une des revendications 1 à 4,
caractérisé
par les étapes opératoires suivantes :
a) déversement, dans un récipient isolé thermiquement, des produits médicinaux conservés dans l'emballage de livraison,
b) obturation du récipient et insufflation/admission du gaz refroidi,
c) séjour des produits médicinaux dans le récipient, jusqu'à ce que la température négative souhaitée se soit établie également au coeur des blocs de produits médicinaux,
d) extraction des produits médicinaux désinsectisés.

6. Procédé d'extermination des vermines, selon la revendication 5,
caractérisé par le fait
que le récipient est une cellule de congélation rapide.

7. Procédé selon la revendication 5,
caractérisé par le fait
que le récipient est un tunnel de refroidissement.

8. Procédé selon la revendication 5,
caractérisé par le fait
que le récipient est un bain d'immersion empli de gaz liquide refroidi.

9. Procédé selon la revendication 5,
caractérisé par le fait
que le récipient est une vis sans fin convoyeuse de refroidissement emplie, dans sa zone de déversement, d un gaz liquide refroidi.

10. Procédé selon la revendication 5,
caractérisé par le fait
qu'un clapet de purge (soupape d'évacuation) installé sur le récipient est fermé, après l'expulsion de l'air provoquée par l'insufflation/l'admission du gaz refroidi, de manière qu'une faible surpression se développe dans ledit récipient.
